# EUROPÄISCHE PATENTSCHRIFT

(11) **EP 1 765 254 B1**
(45) Veröffentlichungstag und Bekanntmachung des Hinweises auf die Patenterteilung: **22.02.2012**
(21) Anmeldenummer: 05744812.8
(22) Anmeldetag: 19.05.2005
(51) Int. Cl.: A61J 1/14

(54) **STERILER PORT**
STERILE PORT
PORT STERILE

(30) Priorität: 09.07.2004 DE 102004033205
(43) Veröffentlichungstag der Anmeldung: 28.03.2007
(73) Patentinhaber: Fresenius Kabi Deutschland GmbH, 61352 Bad Homburg v.d.H. (DE)
(72) Erfinder: KVASNICKA, Annette, 61273 Wehrheim (DE); KNIERBEIN, Bernd, 61267 Neu-Anspach (DE); GRÜNEBERG, Manfred, 61273 Wehrheim (DE)
(86) Internationale Anmeldenummer: PCT/EP2005/005422
(87) Internationale Veröffentlichungsnummer: WO 2006/005391

(56) Entgegenhaltungen:
- EP-A- 0 830 874
- DE-A1- 19 842 960
- DE-B3- 10 313 760

## Beschreibung

Die Erfindung betrifft einen sterilen Port zum Befüllen eines Behälters zur Aufnahme einer enteralen Nährlösung, insbesondere einer flexiblen Verbackung und zum Anschluss einer Schlauchleitung an den Behälter.

Nährlösungen zur enteralen Ernährung werden in Behältern bereitgestellt, die über einen sterilen Port verfügen. Zur Überführung der Nährlösung von dem Behälter zu dem Patienten findet ein sogenanntes Überleitsystem Verwendung, das über eine Schlauchleitung verfügt, die an den sterilen Port angeschlossen wird.

Als Behälter zur Aufnahme von enteralen Nährlösungen sind flexible Beutel bekannt, die aus zwei rechteckförmigen Folienstücken bestehen, die übereinander gelegt und an ihren Rändern miteinander verschweißt sind. Die Nährlösungsbeutel werden automatisch mit enteraler Nährlösung befüllt. Die Befüllung der in Greifern gehaltenen Beutel erfolgt über das offene Kopfende in mehreren Schritten, wobei die Beutel nach dem Befüllen mit den Greifern zusammengezogen und die Verschlussnähte wegen der Schaumbildung infolge der Produktbeteiligung mit Ultraschall geschweißt werden. Über die Verschlussnaht wird zum Verschließen der offenen Restnaht noch eine thermische Kosmetiknaht gesetzt. Bei dieser Art der Befüllung ist der Port grundsätzlich nicht direkt beteiligt.

In der Praxis hat sich gezeigt, dass der bekannte Füll- und Verschließprozess nicht zu konstant befriedigenden Dichtungsergebnissen führt. Dies ist auf die schwer einzuhaltenden Beuteltoleranzen, die chargenbedingten Materialschwankungen, die unterschiedlichen Produkte mit den speziellen Eigenschaften beim Füllprozess, insbesondere der Schaumbildung, und die Komplexität des Füll- und Verschließprozesses zurückzuführen. Deshalb sind in den meisten Fällen aufwendige manuelle Nachkontrollen erforderlich. Um manuelle Nachkontrollen zu vermeiden, sollte der Füll- und Verschließprozess so sicher wie möglich sein.

Die EP-0 830 874 B1 beschreibt einen sterilen Port, der einen anderen als den oben beschriebenen Füll- und Verschließprozess erlaubt. Der Port weist einen in der Art eines Schiffchens ausgebildeten Unterteil und einen Oberteil auf. Der Beutel wird über den offenen Unterteil befüllt, der mit dem Nährlösungsbeutel verschweißt wird. Nach dem Befüllen können Ober- und Unterteil an ihren Flanken miteinander verschweißt werden, so dass der Behälter dicht verschlossen ist. Nachteilig ist, dass die Einbindung eines weiteren Schweißvorganges in den Produktionsprozess relativ aufwendig ist. Allerdings wird mit der bekannten Portfüllung der Formatwechsel der Beutel grundsätzlich vereinfacht.

Aus der DE 103 13 760 ist ein Konnektor für medizinische Flüssigkeiten enthaltende Verpackungen bekannt, dessen Oberteil und Unterteil als einschnappend aufeinander gesetzte Verbindungsstücke ausgebildet sind. Hierzu weist das Oberteil eine umlaufende Nut auf, in die ein umlaufender Vorsprung am Unterteil beim Zusammenpressen von Ober- und Unterteil einschnappt. Mit der einschnappenden Verbindung von Ober- und Unterteil ist zwar der Montageaufwand verringert, nachteilig ist jedoch, dass die Gefahr besteht, dass die Verbindung nicht den beim Gebrauch des Konnektors auftretenden Beanspruchungen standhält.

Neben einer Schnappverbindung schlägt die DE 103 13 760 auch eine zusätzliche Schweißverbindung vor. Auch schlägt die Druckschrift eine keilwellenföxmige Innen- bzw. Außenverzahnung an den zusammengesetzten Teilen vor.

Der Erfindung liegt daher die Aufgabe zugrunde, einen sterilen Port bereitzustellen, der einen Füll- und Verschfießprozess mit hoher Sicherheit, aber verhältnismäßig geringem Aufwand erlaubt.

Die Lösung dieser Aufgabe erfolgt erfindungsgemäß mit den Merkmalen des Patentanspruchs 1. Vorteilhafte Ausführungsformen sind Gegenstand der Unteransprüche,

Darüber hinaus liegt der Erfindung die Aufgabe zugrunde, eine Anordnung mit einem Port und einem Spike zum Anschluss an ein Überleitsystems sowie einen Beutel für enterale Nährlösung mit einem Port zu schaffen, wobei der Port die Sicherheit des Füll- und Verschließprozesses erhöhen und den Prozess vereinfachen soll. Die Lösung dieser Aufgabe erfolgt mit den Ansprüchen 13 bzw. 15.

Bei dem erfindungsgemäßen Port sind der untere Abschnitt des Oberteils und der obere Abschnitt des Unterteils als einschnappend aufeinandergesetzte Verbindungsstücke ausgebildet. Das eine Verbindungsstück weist mindestens einen vorspringenden Ansatz und das andere Verbindungsstück mindestens eine Ausnehmung auf, die den Ansatz passend aufnimmt. Die einschnappend aufeinander gesetzten Verbindungsstücke des Ober- und Unterteils einerseits und der Ansatz und die Ausnehmung der Verbindungsstücke andererseits halten Ober- und Unterteil verdrehsicher zusammen, ohne dass eine zusätzliche Schweißverbindung erforderlich wäre. Die Schnappverbindung zwischen Ober- und Unterteil sollte so ausgelegt sein, dass ein leichtes und zerstörungsfreies Lösen der Verbindung nicht möglich ist. Mit der erfindungsgemäßen Verbindung kann eine hohe Zugfestigkeit und ein hohes Abbrechmoment erzielt werden.

Darüber hinaus weist das Verbindungsstück des Unterteils einen umlaufenden Flansch auf, auf dem mindestens ein vorspringender Ansatz ausgebildet ist. Zur Verdrehsicherung von Ober- und Unterteil weist das Verbindungsstück des Oberteils entsprechend mindestens eine dem mindestens einen Ansatz gegenüberliegende Ausnehmung auf, die den mindestens einen Ansatz passend aufnimmt. Grundsätzlich können nur ein Ansatz und eine Ausnehmung oder mehr als zwei umfaztgsmäßig verteilt angeordnete Ansätze und Ausnehmungen vorgesehen sein.

Bei einer bevorzugten Ausführungsform des erfindungsgemäßen Ports weist das Verbindungsstück des Oberteils einen im wesentlichen zylindrischen oberen Abschnitt auf, an den sich ein nach außen erweiternder Abschnitt anschließt, der in einen im wesentlichen zylindrischen unteren Abschnitt übergeht, wobei der untere Abschnitt einen größeren Innendurchmesser als der obere Abschnitt hat. An der Innenseite des Verbindungsstücks ist eine umlaufende Vertiefung ausgebildet, die einerseits von dem sich nach außen erweiternden Abschnitt und andererseits von einem umlaufenden Vorsprung an der Innenseite des unteren Abschnitts begrenzt wird. Der obere Abschnitt des Verbindungsstücks des Unterteils weist entsprechend an der Innenseite einen umlaufenden Vorsprung auf, der in die umlaufende Vertiefung des Verbindungsstücks des Oberteils passend eingreift,

Bei einer besonders bevorzugten Ausführungstbrm weist der umlaufende Vorsprung des unteren Abschnitts des Verbindungsstücks des Oberteils abgeschrägte Kanten (Einführschräge) auf, so dass sich das Oberteil leichter auf das Unterteil aufsetzen lässt.

Das Verbindungsstück des Oberteils weist zweckmäßigerweise rechtwinklig zueinander angeordnete Seitenflächen auf, so dass sich der Port während des Füll- und Verschließprozesses mit passenden Greifern und Führungsschienen und gegebenenfalls auch zum Abbrechen des Abbrechteils leicht halten lässt.

Um die bekannten Überleitsysteme, die über eine Schraubverbindung verfügen, mit den Port verbinden zu können, ist der obere Abschnitt des Portoberteils vorzugsweise mit einem Außengewinde versehen.

Eine weitere bevorzugte Ausführungsform des Ports zeichnet sich durch eine besonders hohe Dichtigkeit aus. Diese wird dadurch erreicht, dass zwischen Ober- und Unterteil ein Dichtelement klemmend angeordnet ist, das Ober- und Unterteil sicher gegeneinander abdichtet. Die Schnappverbindung zwischen Ober- und Unterteil in Kombination mit dem Dichtelement, das klemmend gehalten wird, ermöglicht eine schnelle, aber dichte Verbindung ohne zusätzlichen Schweißvorgang.

Das Dichtelement dichtet vorzugsweise nicht nur Ober- und Unterteil gegeneinander ab, sondern verschließt auch den Kanal des Ports. Vorteilhafterweise ist das Dichtelement daher als eine von dem Spike der bekannten Überleitsysteme durchstechbare Membran ausgebildet.

Ein besonders sicherer Wiederverscbluss des sterilen Ports nach Abbrechen des Abbrechteils kann dadurch erreicht werden, dass die Membran als Schlitzventil, vorzugsweise aus Kautschuk ausgebildet ist.

Der obere Abschnitt des Portoberteils, der das Aufnahmestück für den Spike bildet, weist vorzugsweise einen kleineren Innendurchmesser als der untere Abschnitt des Oberteils auf. Dadurch ist es möglich, einen Spike mit einem verhältnismäßig kleinen Durchmesser sicher aufzunehmen und zusätzliche Dichtflächen zu bilden.

Eine weitere bevorzugte Ausführungsform sieht einen zweiten umlaufenden Vorsprung an der Innenseite des Verbindungsstücks des Oberteils vor, der oberhalb des ersten umlaufenden Vorsprungs angeordnet ist. Mit diesem Vorsprung wird das Dichtelement gegen Herausrutschen gesichert.

Eine bevorzugte Ausführungsform des erfindungsgemäßen Beutels für enterale Nährlösung zeichnet sich dadurch aus, dass der Port derart angeordnet ist, dass die Längsachse des Ports schräg zu dem Beutelboden, insbesondere in Richtung der unteren Beutelecke verläuft. Dies hat den Vorteil, dass nach dem Einführen des Fülldorns zum Befüllen des Beutels der Füllstrahl den Beutel beim Befüllen öffnet. Vorteilhaft ist auch, dass bei einer derartigen Anordnung des Ports der Beutel sicher ausläuft.

Im Folgenden wird ein Ausführungsbeispiel des sterilen Konnektors zusammen mit den Spike eines Überleitsystems sowie ein Ausführungsbeispiel eines Beutels mit dem erfindungsgemäßen Port unter Bezugnahme auf die Zeichnungen näher erläutert.

Es zeigen:
- Figur 1: eine Konnektoranordnung bestehend aus dem erfindungsgemäßen Port und einem Spike mit Überwurfmutter eines Überleitsystems in geschnittener Darstellung,
- Figur 2: den Ausschnitt A von Figur 1 in vergrößerter Darstellung,
- Figur 3: den Oberteil des Ports mit Abbrechteil in geschnittener Darstellung,
- Figur 4: den Oberteil des Ports von Figur 3 in der Draufsicht,
- Figur 5: eine perspektivische Darstellung des Ober- und Unterteils des Ports und
- Figur 6: eine Seitenansicht eines Nährlösungsbeutels mit Port.

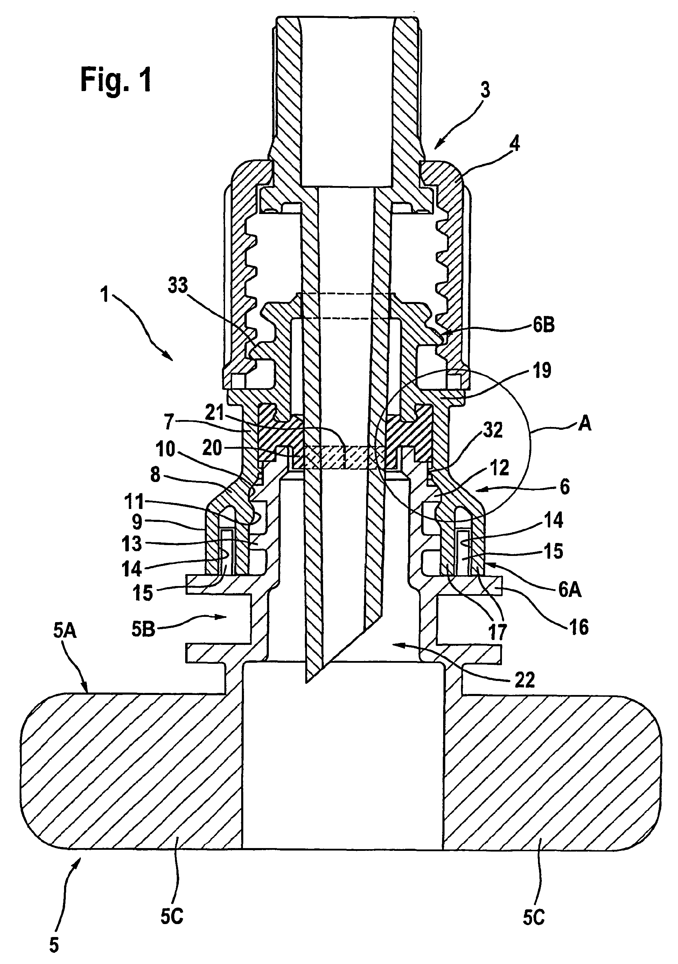

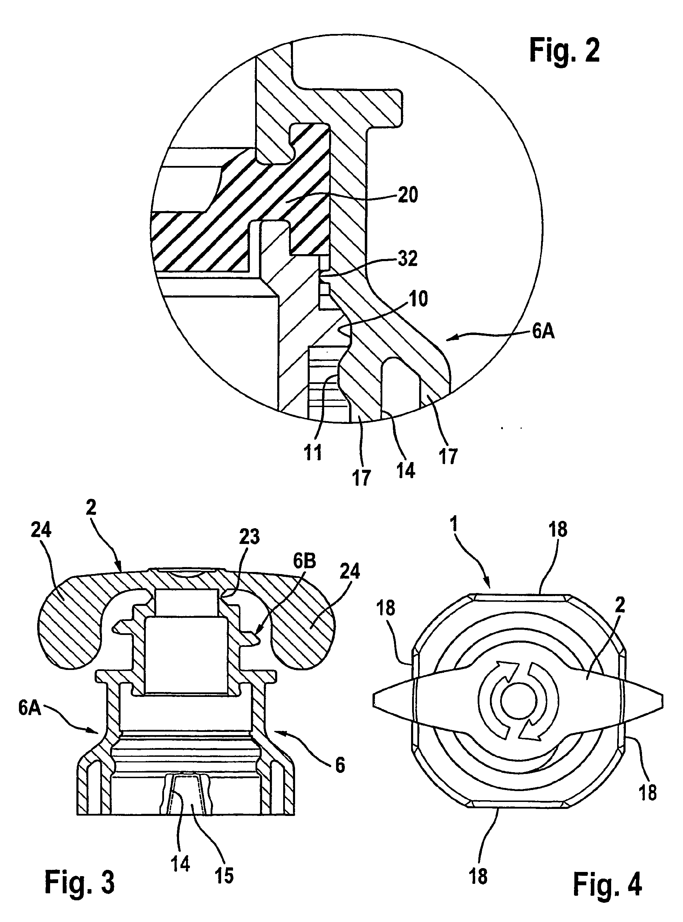

Die Konnektoranordnung umfasst einen sterilen Port 1, der in die Behälterwand einsetzbar und gegenüber der Behälterwand abdichtbar ist, eine abbrechbare Schutzkappe 2 und einen rohrförmigen Einstechdorn 3 mit einer Überwurfmutter 4. Der sterile Konnektor 1 mit abgebrochenem Abbrechteil ist in Figur 1 zusammen mit dem Einstechdorn 3 und der Überwurfmutter 4 dargestellt, während Figur 3 den Port 1 mit Abbrechteil 2 zeigt.

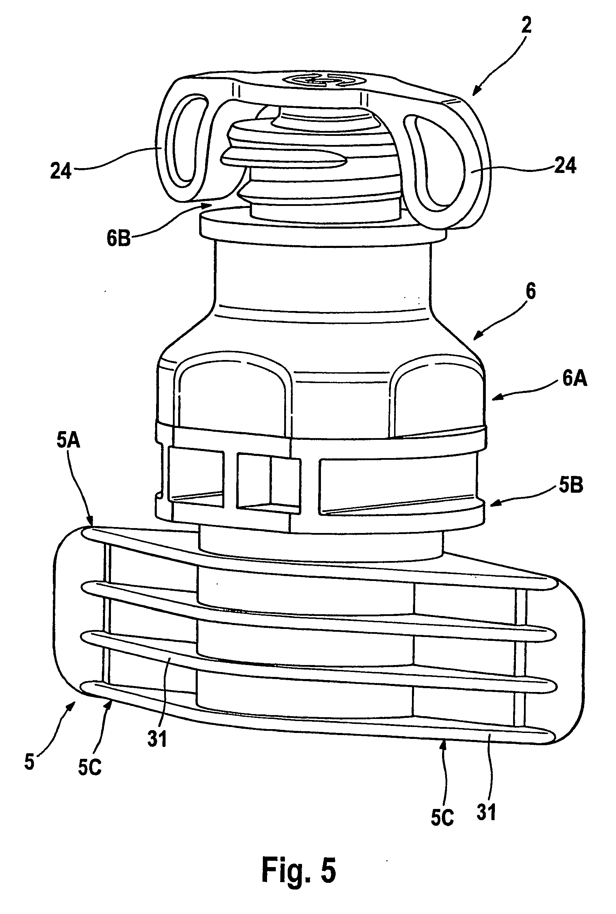

Der sterile Port 1 besteht aus einem Unterteil 5 und einem Oberteil 6, die einschnappend aufeinandergesetzt sind. Der Unterteil 5 des Ports 1 weist einen unteren Abschnitt 5A mit zwei radial abstehenden flügelförmigen Ansätzen 5C auf, die in einer Ebene liegen. Die flügelförmigen Ansätze 5C tragen jeweils vier parallele Rippen 31 (Figur 5), die zu den Enden der Ansätze spitz zulaufen. Der in der Art eines Schiffchen ausgebildete untere Abschnitt 5A des Unterteils 5 kann in die bekannten Folienbeutel für medizinische Flüssigkeiten eingeschweißt werden.

Der im wesentlichen zylindrische obere Abschnitt 5B des Unterteils 5 und der untere Abschnitt 6A des Oberteils 6 des Ports 1 sind als Verbindungsstücke ausgebildet, mit denen Ober- und Unterteil 5, 6 einschnappend miteinander verbunden werden.

Das Verbindungsstück 6A des Oberteils 6 weist einen im wesentlichen zylindrischen oberen Abschnitt 7 auf, der in einen sich nach außen erweiternden Abschnitt 8 übergeht, an den sich ein im wesentlichen zylindrischer unterer Abschnitt 9 anschließt. Der obere Abschnitt 7 weist einen kleineren Innendurchmesser als der untere Abschnitt 9 des Verbindungsstücks 6A auf. Im Bereich des sich nach außen erweiternden Abschnitts 8 ist an der Innenseite des Verbindungsstücks 6A eine umlaufende Vertiefung 10 ausgebildet, an die sich ein umlaufender Vorsprung 11 mit abgeschrägten Kanten an der Innenseite des Verbindungsstücks 6A anschließt.

Das Verbindungsstück 5B des Unterteils 5 weist an der Außenseite einen ersten umlaufenden Vorsprung 12 auf, der einschnappend in der umlaufenden Vertiefung 10 des Verbindungsstücks 6A des Oberteils 6 liegt und sich an dem nach außen erweiternden Abschnitt 8 und dem umlaufendem Vorsprung 11 des Verbindungsstücks abstützt.

Unterhalb des ersten umlaufenden Vorsprungs 11 weist das Verbindungsstück 5B des Unterteils 5 an der Außenseite einen zweiten umlaufenden Vorsprung 13 auf, der unterhalb des umlaufenden Vorsprungs 11 des Verbindungsstücks 6A des Oberteils 6 liegt.

Der Oberteil 6 ist gegenüber dem Unterteil 5 des Ports 1 gegen Verdrehen gesichert. Hierzu weist das Verbindungsstück 6A des Oberteils 6 zwei sich gegenüberliegende Ausnehmungen 15 auf, in die passend zwei Ansätze 15 greifen, die an einen ringförmigen Flansch 16 des Verbindungsstücks 5A des Unterteils 5 angeformt sind. Die Ausnehmungen 14 liegen zwischen seitlichen Stegen 17 in dem doppelwandigen unteren Abschnitt des Verbindungsstücks 6A des Oberteils 6.

Mit der doppelwandigen Ausbildung des Verbindungsstücks wird das Abbrechmoment zwischen Ober- und Unterteil erhöht, wodurch die Festigkeit der Verbindung verbessert wird. Der doppelwandige Abschnitt des Verbindungsstücks 6A weist rechtwinklig zueinander angeordnete Seitenflächen 18 auf, wodurch sich der Port beim Füll- und Verschließprozess und gegebenenfalls auch beim Abbrechen des Abbrechteils 2 besser greifen lässt (Figur 4).

Der obere Abschnitt des Oberteils des Ports 1 ist als Aufnahmestück 6B für einen Spike 3 eines nicht dargestellten Überleitsystems ausgebildet. Das im wesentlichen zylindrische Aufnahmestück 6B des Oberteils 6 weist einen kleineren Innendurchmesser als das Verbindungsstück 6A des Oberteils auf, wobei das Aufnahmestück 6B über einen nach innen weisenden Ansatz 19 an dem Verbindungsstück 6A angeformt ist.

Der Oberteil 6 ist gegenüber dem Unterteil 5 des Ports 1 mit einem tellerförmigen Dichtelement 20 abgedichtet, das zwischen den Verbindungsstücken des Ober- und Unterteils klemmend angeordnet ist (Figur 2). Das Dichtelement ist eine Membran 20, die im Zentrum ein nur andeutungsweise dargestelltes Schlitzventil 21 aufweist, so dass die Membran leicht von dem Spike 3 durchstochen werden kann, die Membran aber den Kanal 22 des Ports 1 nach Herausziehen des Spikes dicht verschließt. Eine zusätzliche Abdichtung schafft ein umlaufender Vorsprung 23 an der Innenseite des Verbindungsstücks 6A des Oberteils 6, der an der Außenseite des zylindrischen oberen Abschnitts des Verbindungsstücks 5B des Unterteils 5 dicht anliegt. Das Aufnahmestück 6B des Oberteils 6 weist ein Außengewinde 33 auf, so dass die Überwurfmutter 4 des Spikes 3 mit dem Port verschraubt werden kann.

Figur 3 zeigt den Oberteil 6 des in einen enteralen Nährlösungsbeutel einzuschweißenden Port 1 ohne Spike und Überwurfmutter. Der Kanal 22 des Ports ist mit einem Abbrechteil 2 verschlossen, der über eine Ringbruchzone 32 an das Aufnahmestück 6B des Ports angeformt ist. Der Abbrechteil 2 weist zwei radial abstehende Flügel 24 auf, um den Abbrechteil besser greifen zu können. Der Abbrechteil 2 bildet gleichsam einen Originalitätsverschluss.

Ober- und Unterteil 6A, 6B des Ports 1 bestehen aus thermoplastischen Kunststoffen, vorzugsweise Polyolefinen, wie Polypropylen. Sie können im Spritzgussverfahren hergestellt werden. Die durchstechbare Membran 20 besteht aus Kautschuk.

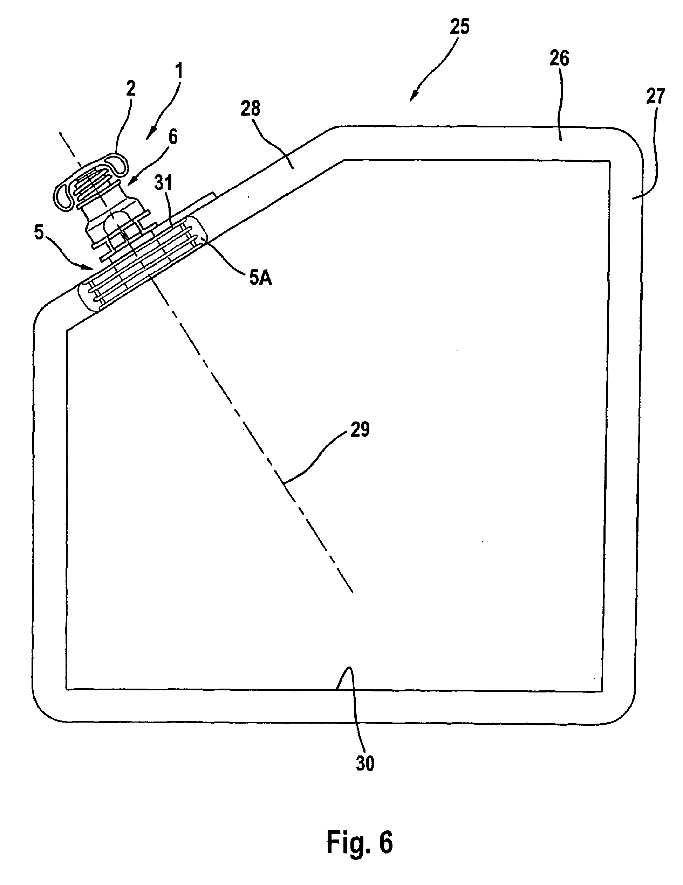

Figur 6 zeigt einen Folienbeutel 25, der mit einer enteralen Nährlösung befüllt und mit dem Port 1 verschlossen ist. Der Beutel 25 besteht aus zwei übereinander liegenden Folien 26, die an ihren Rändern 27 miteinander verschweißt sind. Der in der Art eines Schiffchens ausgebildete untere Abschnitt 5A des Unterteils 5 des Ports 1 liegt zwischen den beiden Folienlagen und ist mit den Folien dicht verschweißt. Der Port ist in einem abgeschrägten Abschnitt der oberen Schweißnaht 28 derart angeordnet, dass dessen Längsachse 29 auf den Beutelboden 30 zeigt.

Während des Füll- und Verschließprozesses ist der Oberteil des Ports 1 nicht auf den in den Beutel 25 eingeschweißten Unterteil 5 aufgesetzt. Der Beutel 25 wird mittels eines in den Kanal 22 des Portunterteils 5 eingeführten Fülldorns automatisch mit der enteralen Nährlösung abdichtend befüllt. Anschließend wird der Oberteil 6 zusammen mit der Membran 20 auf den Unterteil 5 aufgesetzt. Damit ist der Port 1 dicht verschlossen.

Die Verbindung von Portober- und unterteil kann in einem automatisierten Prozess erfolgen. Hierzu werden die Beutel in einer Füllstation an dem Portunterteil gehalten, während die Beutel um eine zentrale Achse rotieren. Die Portoberteile werden in einem ersten Schritt kontinuierlich auf die rotierenden Portunterteile zunächst nur lose aufgesetzt. Da Portober- und -unterteil an den Anlageflächen abgeschrägte Kanten aufweisen, zentriert sich der Oberteil auf dem Unterteil. In einem zweiten Schritt werden dann die Oberteile auf die Unterteile fest aufgedrückt.

Kurz vor Gebrauch wird der Port durch Abdrehen des Abbrechteils 2 geöffnet. Dann kann der Spike 3 des Überleitsystems in den Portkanal 22 eingeschoben und der Spike mit der Überwurfmutter 4 mit dem Port verschraubt werden. Dabei wird die Membran 20 von dem Spike durchstochen. Nunmehr kann die enterale Nährlösung dem Beutel entnommen werden.

Beim Befüllen öffnet der auf den Beutelboden 30, insbesondere die untere Beutelecke gerichtete Füllstrahl den Beutel. Der Beutel wird nicht vollständig befüllt, so dass ein Kopfraum (Luftblase) frei bleibt. Der für die enterale Ernährung notwendige Kopfraum führt beim freihängenden Beutel dazu, dass nach Lösen des Fülldorns vom Port der Beutel unverzüglich belüftet wird und nach unten wegsackt. Dadurch wird auch das Produkt bzw. der Schaum von der Füllöffnung weggezogen.

## Patentansprüche

1. Steriler Port zum Anschluss einer Schlauchleitung an einen Behälter zur Aufnahme einer enteralen Nährlösung mit
einem in den Behälter einsetzbaren und gegenüber dem Behälter abdichtbaren Unterteil (5) mit einem Kanal (22), einem auf dem Unterteil sitzenden Oberteil (6) mit einem Kanal (22) und einem auf dem Oberteil sitzenden Abbrechteil (2), der den Kanal des Ports verschließt, wobei der obere Abschnitt des Oberteils (6) als Aufnahmestück (6B) ausgebildet ist, in den ein mit der Schlauchleitung verbundener Spike zur Entnahme der Flüssigkeit einsetzbar ist, wobei der untere Abschnitt des Oberteils (6) und der obere Abschnitt des Unterteils (5) als einschnappend auieinaudergesetzte Verbindungsstücke (6A, 5B) ausgebildet sind, **dadurch gekennzeichnet, dass** das Verbindungsstück (5B) des Unterteils (5) einen Flansch (16) aufweist, auf dem mindestens ein vorspringender Ansatz (15) ausgebildet ist, wobei das Verbindungsstück (6A) des Oberteils (6) mindestens eine dem mindestens einen Ansatz (15) gegenüberliegende Ausnehmung (14) aufweist, die den mindestens einen Ansatz (15) zur Verdrehsicherung passend aufnimmt.

2. Steriler Port nach Anspruch 1, **dadurch gekennzeichnet, dass** das Verbindungsstück (6A) des Oberteils (6) einen im wesentlichen zylindrischen oberen Abschnitt (7) aufweist, an den sich ein nach außen erweiternder Abschnitt (8) anschließt, der in einen im wesentlichen zylindrischen unteren Abschnitt (9) übergeht, der einen größeren Innendurchmesser als der obere Abschnitt hat, wobei an der Innenseite des Verbindungsstücks eine umlaufende Vertiefung (10) ausgebildet ist, die einerseits von dem sich nach außen erweiternden Abschnitt (8) und andererseits von einem umlaufenden Vorsprung (11) an der Innenseite des unteren Abschnitts (9) begrenzt wird.

3. Steriler Port nach Anspruch 2, **dadurch gekennzeichnet, dass** der umlaufende Vorsprung (11) des unteren Abschnitts (9) des Verbindungsstücks (6A) des Oberteils (6) abgeschrägte Kanten aufweist.

4. Steriler Port nach einem der Ansprüche 1 bis 3, **dadurch gekennzeichnet, dass** das Verbindungsstück (5B) des Unterteils (5) an der Außenseite einen zweiten umlaufenden Vorsprung (13) aufweist, der unterhalb des ersten umlaufenden Vorsprungs (12) angeordnet ist.

5. Steriler Port nach einem der Ansprüche 1 bis 4, **dadurch gekennzeichnet, dass** auf dem Flansch (16) des Verbindungsstücks (5B) des Unterteils (5) an einander gegenüberliegenden Seiten zwei vorspringende Ansätze (15) ausgebildet sind, wobei das Verbindungsstück (6A) des Oberteils (6) zwei den Ansätzen gegenüberliegende Ausnehmungen (14) aufweist, die die Ansätze passend aufnehmen.

6. Steriler Port nach einem der Ansprüche 1 bis 5, **dadurch gekennzeichnet, dass** das Verbindungsstück (6A) des Oberteils (6) rechtwinklig zueinander angeordnete Seitenflächen (18) aufweist.

7. Steriler Port nach einem der Ansprüche 1 bis 6, **dadurch gekennzeichnet, dass** das Aufnahmestück (6B) des Oberteils (6) mit einem Außengewinde (24) versehen ist.

8. Steriler Port nach einem der Ansprüche 1 bis 7, **dadurch gekennzeichnet, dass** zwischen dem Oberteil (6) und dem Unterteil (5) ein Oberteil gegenüber Unterteil abdichtendes Dichtelement (20) klemmend angeordnet ist.

9. Steriler Port nach Anspruch 8, **dadurch gekennzeichnet, dass** das Dichtelement als den Kanal (22) des Ports verschließende Membran (20) ausgebildet ist, die von dem Spike durchstechbar ist.

10. Steriler Port nach Anspruch 9, **dadurch gekennzeichnet, dass** die Membran (20) als Schlitzventil, vorzugsweise aus Kautschuk ausgebildet ist.

11. Steriler Port nach einem der Ansprüche 1 bis 10, **dadurch gekennzeichnet, dass** das Aufnahmestück (6B) des Oberteils (6) einen kleineren Innendurchmesser als das Verbindungsstück (6A) des Oberteils (6) aufweist, wobei das Aufnahmestück über einen nach innen weisenden Ansatz (19) an dem Verbindungsstück befestigt ist,

12. Steriler Port nach einem der Ansprüche 9 bis 11, **dadurch gekennzeichnet, dass** der obere Abschnitt (7) des Verbindungsstücks (6A) des Oberteils (6) an der Innenseite einen umlaufenden Vorsprung (23) aufweist.

13. Anordnung mit einem Port (1) nach einem der Ansprüche 1 bis 12 und einem Spike (3) zum Anschluss an ein Überleitsystem.

14. Anordnung nach Anspruch 13, **dadurch gekennzeichnet, dass** der Spike (3) eine Überwurfmutter (4) aufweist, die auf das mit einem Außengewinde (24) versehene Aufnahmestück (6B) des Oberteils (6) des Ports (1) aufschraubbar ist.

15. Beutel für enterale Nährlösung mit einem Port (1) nach einem der Ansprüche 1 bis 12 oder einer Anordnung nach einem der Ansprüche 13 oder 14.

16. Beutel nach Anspruch 15, **dadurch gekennzeichnet, dass** der Port (1) derart angeordnet ist, dass die Längsachse (29) des Ports schräg zu dem Beutelboden (30) verläuft.

## Claims

1. A sterile port for the connection of a hose line to a container for accommodating an enteral nutritive solution with
a lower part (5) with a conduit (22), which lower part can be inserted into the container and sealed with respect to the container, an upper part (6) with a conduit (22), which upper part sits on the lower part, and a snap-off part (2) which sits on the upper part and seals the conduit of the port, wherein the upper portion of the upper part (6) is constituted as a receiving piece (6B), into which a spike connected to the hose line can be inserted for the removal of the fluid, wherein the lower portion of the upper part (6) and the upper portion of the lower part (5) are constituted as connecting pieces (6A, 5B) placed one upon the other in a snap-in fashion, **characterised in that** the connecting piece (5B) of the lower part (5) comprises a flange (16), on which at least one projecting extension (15) is formed, wherein the connecting piece (6A) of the upper part (6) comprises at least one recess (14) lying opposite the at least one extension (15), said recess receiving the at least one extension (15) in order to secure against rotation.

2. The sterile port according to claim 1, **characterised in that** the connecting piece (6A) of the upper part (6) comprises an essentially cylindrical upper portion (7), which is followed by an outwardly extending portion (8), which transforms into an essentially cylindrical lower portion (9), which has a larger internal diameter than the upper portion, wherein there is constituted on the inside of the connecting piece a circumferential recess (10), which on the one hand is bounded by the outwardly extending portion (8) and on the other hand by a circumferential projection (11) on the inside of the lower portion (9).

3. The sterile port according to claim 2, **characterised in that** the circumferential projection (11) of the lower portion (9) of the connecting piece (6A) of the upper part (6) has bevelled edges.

4. The sterile port according to any one of claims 1 to 3, **characterised in that** the connecting piece (5B) of the lower part (5) has on the outside a second circumferential projection (13) which is disposed beneath the first circumferential projection (12).

5. The sterile port according to any one of claims 1 to 4, **characterised in that** two projecting extensions (15) are formed on opposite sides on the flange (16) of the connecting piece (5B) of the lower part (5), wherein the connecting piece (6A) of the upper part (6) comprises two recesses (14) lying opposite the extensions, said recesses receiving the extensions in a matching fashion.

6. The sterile port according to any one of claims 1 to 5, **characterised in that** the connecting piece (6A) of the upper part (6) comprises side faces (18) disposed at right angles to one another.

7. The sterile port according to any one of claims 1 to 6, **characterised in that** the receiving piece (6B) of the upper part (6) is provided with an external thread (24).

8. The sterile port according to any one of claims 1 to 7, **characterised in that** a sealing element (20) is disposed in a clamped fashion between the upper part (6) and the lower part (5), said sealing element sealing the upper part with respect to the lower part.

9. The sterile port according to claim 8, **characterised in that** the sealing element is constituted as a membrane (20) which seals the conduit (22) of the port and can be pierced by a spike.

10. The sterile port according to claim 9, **characterised in that** the membrane (20) is constituted as a slot valve, preferably made of rubber.

11. The sterile port according to any one of claims 1 to 10, **characterised in that** the receiving piece (6B) of the upper part (6) has a smaller internal diameter than the connecting piece (6A) of the upper part (6), wherein the receiving piece is fixed by means of an inwardly pointing extension (19) to the connecting piece.

12. The sterile port according to any one of claims 9 to 11, **characterised in that** the upper portion (7) of the connecting piece (6A) of the upper part (6) comprises a circumferential extension (23) on the inside.

13. An arrangement with a port (1) according to any one of claims 1 to 12 and a spike (3) for the connection to a transfer system.

14. The arrangement according to claim 13, **characterised in that** the spike (3) comprises a union nut (4), which can be screwed onto the receiving piece (6B) of the upper part (6) of the port (1), said receiving piece being provided with an external thread (24).

15. A bag for enteral nutritive solution with a port (1) according to any one of claims 1 to 12 or an arrangement according to any one of claims 13 or 14.

16. The bag according to claim 15, **characterised in that** the port (1) is disposed in such a way that the longitudinal axis (29) of the port runs obliquely towards the bag bottom (30).

## Revendications

1. Port stérile pour raccordement à une conduite tubulaire d'un conteneur destiné à recevoir une solution nutritive entérale comportant
une partie inférieure (5) à insérer dans le conteneur et pouvant éventuellement être scellée par rapport au conteneur, dotée d'un canal (22), une partie supérieure (6) placée sur la partie inférieure dotée d'un canal (22) et une partie à casser (2) placée sur la partie supérieure, qui ferme le canal du port, le segment supérieur de la partie supérieure (6) étant conçu comme un élément de réception (6B), dans lequel une pointe reliée à la conduite tubulaire est utilisable pour prélever du liquide, le segment inférieur de la partie supérieure (6) et le segment supérieur de la partie inférieure (5) étant conçus comme des éléments de raccordement (6A, 5B) insérés l'un sur l'autre par encliquetage, **caractérisé en ce que** l'élément de raccordement (5B) de la partie inférieure (5) présente une bride (16) sur laquelle au moins un ergot proéminent (15) est constitué, l'élément de raccordement (6A) de la partie supérieure (6) présentant au moins un évidement (14) opposé à au moins un ergot (15) qui reçoit de façon adaptée au moins l'ergot (15) pour former un système anti-rotation.

2. Port stérile selon la revendication 1, **caractérisé en ce que** l'élément de raccordement (6A) de la partie supérieure (6) présente un segment supérieur (7) substantiellement cylindrique sur lequel un segment s'élargissant vers l'extérieur (8) est emboîté, qui passe dans un segment inférieur (9) substantiellement cylindrique qui a un diamètre intérieur supérieur à celui du segment supérieur, dans lequel sur le côté intérieur de l'élément de raccordement, une cannelure (10) périphérique est formée, qui est limitée d'une part par un segment s'élargissant vers l'extérieur (8) et d'autre part, par un bossage périphérique (11) sur le côté intérieur du segment inférieur (9).

3. Port stérile selon la revendication 2, **caractérisé en ce que** le bossage périphérique (11) du segment inférieur (9) de l'élément de raccordement (6A) de la partie supérieure (6) présente des bords biseautés.

4. Port stérile selon l'une des revendications 1 à 3, **caractérisé en ce que** l'élément de raccordement (5B) de la partie inférieure (5) présente du côté extérieur un deuxième bossage périphérique (13) qui est disposé sous le premier bossage périphérique (12).

5. Port stérile selon l'une des revendications 1 à 4, **caractérisé en ce que** sur la bride (16) de l'élément de raccordement (5B) de la partie inférieure (5) sont formés sur deux côtés opposés, deux ergots proéminents (15) l'élément de raccordement (6A) de la partie supérieure (6) présentant deux évidements (14) opposés aux ergots qui reçoivent les ergots de façon adaptée.

6. Port stérile selon l'une des revendications 1 à 5, **caractérisé en ce que** l'élément de raccordement (6A) de la partie supérieure (6) présente des faces latérales (18) disposées à angle droit les unes par rapport aux autres.

7. Port stérile selon l'une des revendications 1 à 6, **caractérisé en ce que** l'élément de réception (6B) de la partie supérieure (6) est doté d'un filetage extérieur (24).

8. Port stérile selon l'une des revendications 1 à 7, **caractérisé en ce que**, entre la partie supérieure (6) et la partie inférieure (5) est disposé un élément d'étanchéité (20) assurant l'étanchéité de la partie supérieure par rapport à la partie inférieure.

9. Port stérile selon la revendication 8, **caractérisé en ce que** l'élément d'étanchéité est conçu comme la membrane (20) fermant le canal (22) du port, qui peut être perforée par la pointe.

10. Port stérile selon la revendication 9, **caractérisé en ce que** la membrane (20) est conçue comme une valve fendue, de préférence en caoutchouc.

11. Port stérile selon l'une des revendications 1 à 10, **caractérisé en ce que** l'élément de réception (6B) de la partie supérieure (6) présente un diamètre intérieur plus petit que celui de l'élément de raccordement (6A) de la partie supérieure (6), l'élément de réception étant fixé à l'élément de raccordement sur un ergot (19) tourné vers l'intérieur.

12. Port stérile selon l'une des revendications 9 à 11, **caractérisé en ce que** le segment supérieur (7) de l'élément de raccordement (6A) de la partie supérieure (6) présente sur le côté intérieur, un bossage périphérique (23).

13. Dispositif comportant un port (1) selon l'une des revendications 1 à 12 et une pointe (3) pour le raccordement à un système de transition.

14. Dispositif selon la revendication 13, **caractérisé en ce que** la pointe (3) présente un écrou-raccord (4) qui peut être vissé sur l'élément de réception (6B) de la partie supérieure (6) du port (1), lequel élément de réception est pourvu d'un filetage extérieur (24).

15. Poche pour solution nutritive entérale comportant un port (1) selon l'une des revendications 1 à 12, ou comportant un dispositif selon l'une des revendications 13 ou 14.

16. Poche selon la revendication 15, **caractérisée en ce que** le port (1) est disposé de telle sorte que l'axe longitudinal (29) du port s'étend perpendiculairement par rapport au fond de la poche (30).
